# EUROPEAN PATENT SPECIFICATION

(11) **EP 4 087 526 B1**
(45) Date of publication and mention of the grant of the patent: **04.03.2026**
(21) Application number: 20912405.6
(22) Date of filing: 18.12.2020
(51) Int. Cl.: A61G 13/10, A61B 46/10, A61B 90/40, F24F 8/00, A61B 50/00, F24F 8/10, F24F 8/20

(54) **A SCREEN COVER DEVICE FOR AN AIR-CLEANING DEVICE**
SIEBABDECKVORRICHTUNG FÜR EINE LUFTREINIGUNGSVORRICHTUNG
DISPOSITIF DE RECOUVREMENT DE GRILLE POUR DISPOSITIF D'ÉPURATION D'AIR

(30) Priority: 07.01.2020 SE 2050006
(43) Date of publication of application: 16.11.2022
(73) Proprietor: Toul Meditech AB, 721 33 Västerås (SE)
(72) Inventor: HANSSON, Tomas, 725 97 VÄSTERÅS (SE)
(74) Representative: Brann AB
(86) International application number: PCT/SE2020/051231
(87) International publication number: WO 2021/141522

(56) References cited:
- EP-A1- 1 958 568
- WO-A1-2018/201186
- CA-A1- 2 977 955
- CN-A- 106 309 074
- CN-Y- 2 886 398
- JP-A- H04 250 808
- KR-B1- 101 963 804
- US-A- 4 045 192
- US-A1- 2008 216 844
- US-A1- 2009 233 540
- US-A1- 2011 005 178
- US-A1- 2013 263 868
- US-B2- 6 811 593
- US-B2- 6 811 593

## Description

### TECHNICAL FIELD OF THE INVENTION

The present invention relates to screen covers for an air-cleaning device. It furthermore relates to an air-cleaning device system.

### BACKGROUND

Air-cleaning devices are useful in sensitive environments such as for example operating rooms, drug manufacture, micro-chip production and similar areas. Movable air-cleaning devices are for example described in US6811593. Such air-cleaning devices can for example be positioned in front of an operating table or an instrument table for providing a clean air flow over the table. A single use screen cover is sometimes provided over a screen of the air-cleaning device. Hereby the risk that the staff working during the surgery will destroy their sterility by touching the air-cleaning device is reduced since touching the single-use screen cover would not be a problem. A single use screen cover may be provided for improving sterility and for providing a laminar air flow.

### SUMMARY

An object of the invention is to provide improved disposable screen covers for air-cleaning devices.

A further object of the invention is to provide user-friendly screen covers.

This is achieved by a screen cover device and an air cleaning device system according to the independent claims.

According to one aspect of the invention a screen cover device is provided for holding disposable screen covers which when in use are configured for covering at least a substantial part of an air outflow area of a screen of an air-cleaning device, wherein said screen cover device is provided as an attachment to the air-cleaning device and comprises:
- at least two disposable screen covers; and
- a first unit which is configured for keeping the at least two disposable screen covers when unused and which is configured to be mounted to the air-cleaning device,
   wherein said at least two disposable screen covers (7) are in connection with each other such that they can be removed from the first unit (13) one after each other.

According to another aspect of the invention an air-cleaning device system comprising an air-cleaning device and a screen cover device as described above is provided, wherein said air-cleaning device (1) comprises:
- a screen (11) with an air outflow area (9) where clean air is flowed out from the air-cleaning device (1); and
- a first screen cover device holder (51a) for holding said first unit (13.

Hereby a screen cover device is provided which comprises at least two disposable screen covers. The disposable screen covers can be provided to the air-cleaning device one after the other, i.e. a new one for each use. Hereby a user-friendly device is provided which can be attached to an air-cleaning device. Hereby sterility in for example an operation room, a surgery preparation room, another type of health care room or another room where sterility is needed can be improved.

In one embodiment of the invention the first unit comprises a roll around which the at least one unused disposable screen cover is rolled.

In one embodiment of the invention a first end of each disposable screen cover is configured for being connected to the air-cleaning device, whereby a second end of the disposable screen cover is held by the first unit when this disposable screen cover is the one presently in use covering an air outflow area of a screen of the air-cleaning device, which second end is opposite said first end of the disposable screen cover.

In one embodiment of the invention the screen cover device further comprises:
- a second unit for collecting used disposable screen covers, which second unit is configured to be mounted to the air-cleaning device such that a screen cover which is the one presently in use for covering at least a substantial part of an air outflow area of a screen of the air-cleaning device is held between the first and the second units.

In one embodiment of the invention the second unit comprises a roll around which the used disposable screen covers are rolled.

In one embodiment of the invention the rolls of the first and second units are configured such that they can be rotated in coordination for unwinding unused disposable screen covers from the roll of the first unit and winding used disposable screen covers onto the roll of the second unit.

In one embodiment of the invention the disposable screen covers are designed to cover at least a substantial part of the air outflow area of the screen of the air-cleaning device and at least parts of side edges of the air-cleaning device and wherein the screen cover device is configured to be mounted to the air-cleaning device such that one of the disposable screen covers which is the one to be presently in use covers both at least a substantial part of the air outflow area of the screen of the air-cleaning device and at least parts of side edges of the air-cleaning device.

In one embodiment of the invention at least one of the disposable screen covers comprises a peelable protective layer.

In one embodiment of the invention the screen cover device is presterilized.

In one embodiment of the invention the air-cleaning device further comprises a second screen cover device holder for holding a second unit of a screen cover device or for holding a first end of a screen cover.

In one embodiment of the invention said second screen cover device holder is positioned such that a screen cover can be held in a first end either by a second unit or by the second screen cover device holder and in a second end by the first unit while the screen cover is covering at least a substantial part of the air outflow area of the screen of the air-cleaning device.

In one embodiment of the invention the air-cleaning device further comprises a germicidal radiation source positioned such that the disposable screen cover to be used can be irradiated.

In one embodiment of the invention said germicidal radiation source is provided such that it can irradiate the disposable screen cover which presently is in use and the air outflow from the air-cleaning device.

In one embodiment of the invention the air-cleaning device further comprises a frame configured to hold the one of the disposable screen covers which is the one to be presently in use in a position such that the disposable screen cover covers at least a substantial part of the air outflow area of the screen of the air-cleaning device.

In one embodiment of the invention the frame comprises guides which are bent such that the screen cover which is the one presently used is stretched over the guides and provides a curved surface of the screen cover.

### BRIEF DESCRIPTION OF THE DRAWINGS

Figures 1a and 1b are two different perspective views of an air-cleaning device provided with a screen cover device according to one embodiment of the invention.
Figure 2 shows an air-cleaning device provided with a screen cover device according to another embodiment of the invention.
Figure 3 is a perspective view from above of an air-cleaning device provided with a screen cover device according to another embodiment of the invention.

### DETAILED DESCRIPTION OF THE EMBODIMENTS

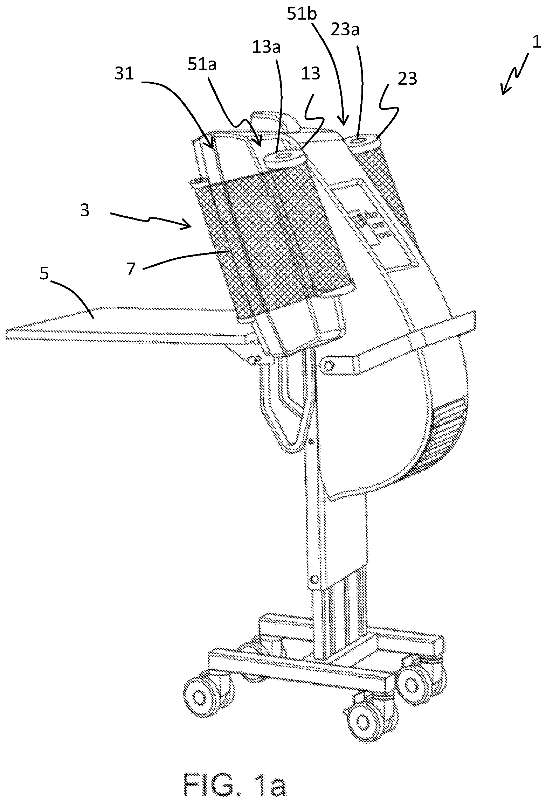

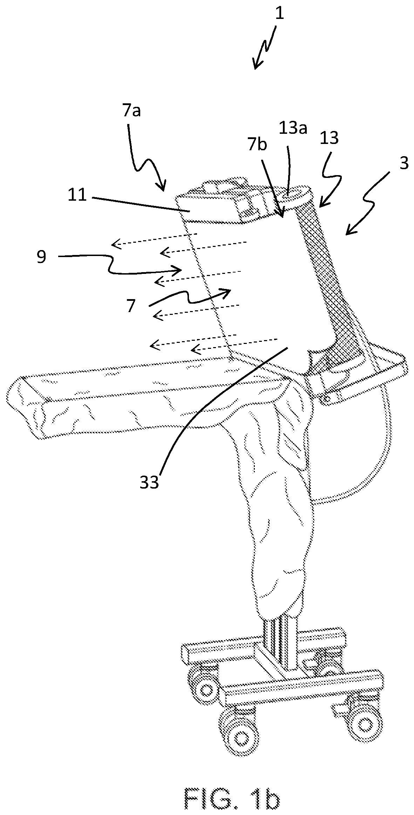

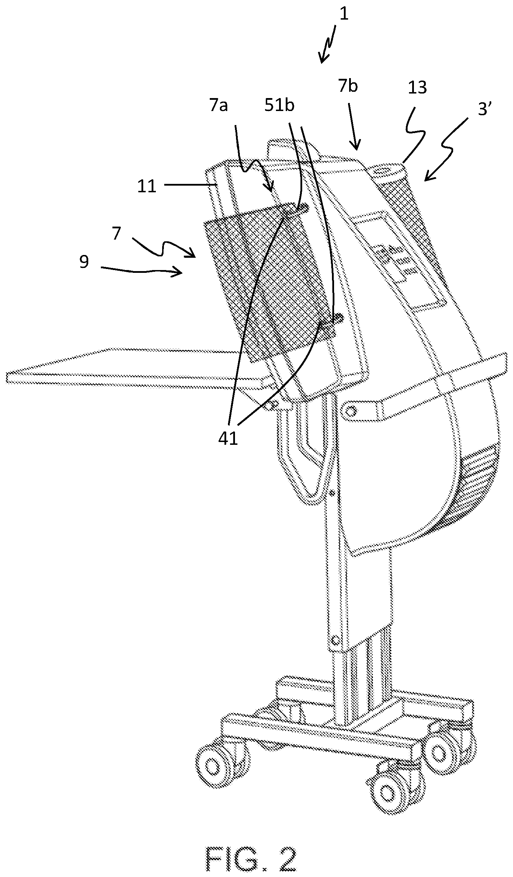

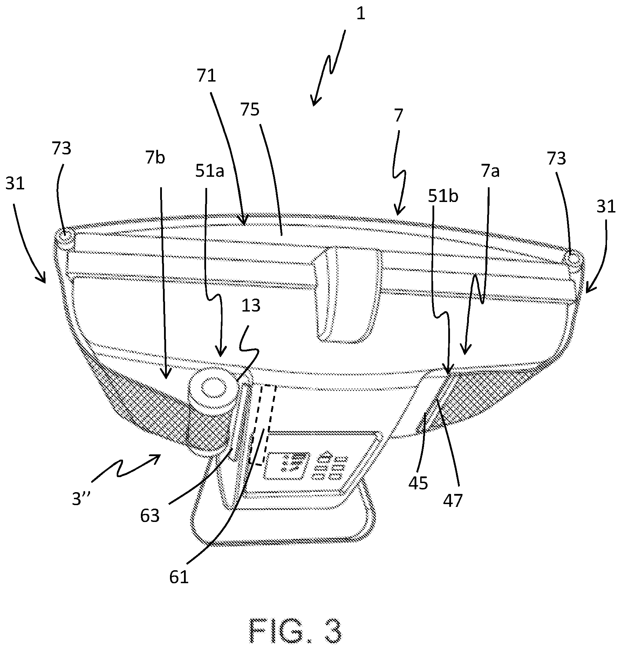

Figures 1a and 1b are two different perspective views of an air-cleaning device 1 provided with a screen cover device 3 according to one embodiment of the invention. Figure 2 shows an air-cleaning device 1 provided with a screen cover device 3' according to another embodiment of the invention and Figure 3 is a perspective view from above of an air-cleaning device 1 provided with a screen cover device 3" according to still another embodiment of the invention.

Some of the details in these three different embodiments are the same or similar and are also given the same reference numbers and will be described together with reference to all the drawings. The air-cleaning device 1 is in this illustration a movable air-cleaning device 1 which suitably can be used in operating rooms. The air-cleaning device is here provided with a table 5 which can be fixed, foldable or removable. However, the air-cleaning device used in this invention does not need to have a table but can instead be positioned in front of an operating table or an instrument table for providing a clean air flow over the table. The air-cleaning device as used in this invention does not have to be movable but can instead be mounted to for example an operating table or an instrument table. It can instead also be provided to a wall mounted or ceiling mounted adjustable stand or frame allowing the air-cleaning device to be moved into different positions within a room.

According to the invention a screen cover device 3 is provided for holding at least one disposable screen covers 7 or a number of disposable screen covers 7 which when in use are configured for covering at least a substantial part of an air outflow area 9 of a screen 11 of the air-cleaning device 1. A substantial part of the air outflow area 9 would mean for example more than 75% of the air outflow area 9 or more than 90% of the air outflow area. According to the invention said screen cover device 3 is provided as an attachment to the air-cleaning device 1 and comprises a first unit 13 which is configured for holding at least one unused disposable screen cover 7 and which is configured to be mounted to the air-cleaning device 1. The one or more disposable screen covers 7 are before use kept or stored within the first unit 13 and the screen cover device 3 is configured for holding the disposable screen cover 7 which is presently in use for covering at least a substantial part of an air outflow area 9 of a screen 11 of an air-cleaning device. The disposable screen covers 7 provided in the screen cover device 3 are used one after each other for covering at least a substantial part of an air outflow area 9 of a screen 11 of an air-cleaning device. The screen cover device 3 comprises at least one disposable screen cover 7. The screen cover device 3 comprises suitably at least two disposable screen covers 7 which are in connection with each other such that they can be removed from the first unit 13 one after each other.

In all the embodiments as shown in Figures 1-3 the first unit 13 comprises a roll 13a around which the at least one unused disposable screen cover 7 is rolled. However, other alternatives than rolling the screen covers 7 around a roll are possible within the scope of the invention. For example, the first unit could instead be a cassette within which the screen covers 7 are folded.

In the embodiment of the invention as shown in Figures 1a and 1b the screen cover device 3 further comprises a second unit 23 for collecting used disposable screen covers 7, which second unit 23 is configured to be mounted to the air-cleaning device 1 such that a screen cover 7 which is the one presently in use for covering at least a substantial part of an air outflow area 9 of a screen 11 of the air-cleaning device 1 is held between the first unit 13 and the second unit 23. In this embodiment the second unit 23 comprises a roll 23a around which the used disposable screen covers 7 are rolled. However, in another embodiment the second unit 23 could as well be another type of cassette in which used screen covers 7 can be collected also in other ways than rolling them.

In a screen cover device 3 according to the embodiment as shown in Figures 1a and 1b the rolls 13a, 23a of the first and second units 13, 23 can be configured such that they can be rotated in coordination for unwinding unused disposable screen covers 7 from the roll 13a of the first unit 13 and winding used disposable screen covers 7 onto the roll 23a of the second unit 23. This can be done both manually by rotating one of the rolls 13a, 23a or powered by a motor. Hereby a new disposable screen cover 7 can easily be provided for, for example each new operation. The whole screen cover device 3 with its unused screen covers 7 may have been pre-sterilized before mounting to the air-cleaning device 1 and maybe even before delivery and packed in a sterile package for assuring a sterile product. The disposable screen covers 7 provided within the screen cover device 3 may also be provided with one or more protection films provided between each of the screen covers 7 for covering a connection boundary between the screen covers and possibly an area around the connection boundary. This protection film should protect the next following screen cover from a previous already used screen cover 7 and the protection film should not be removed until the previous screen cover 7 has been collected safely according to sterile routines. Hereby a risk for contamination from a previous operation can be avoided.

In the embodiments shown in Figure 2 and Figure 3 the screen cover device 3'; 3" does not comprise a second unit. Instead a first end 7a of each disposable screen cover 7 is configured for being connected to the air-cleaning device 1, whereby a second end 7b of the disposable screen cover 7 is held by the first unit 13 when this disposable screen cover 7 is the one presently in use covering an air outflow area 9 of a screen 11 of the air-cleaning device 1, which second end 7b is opposite said first end 7a of the disposable screen cover 7. The first end 7a of each disposable screen cover 7 comprises in some embodiments of the invention an air-cleaning device connector 41 for connection to a second screen cover device holder 51b provided on the air-cleaning device 1. A first screen cover device holder 51b is provided on an opposite side of the air-cleaning device 1 and is configured for holding the first unit 13 of the screen cover device 3; 3'; 3" which will be described further below. In the example as shown in Figure 2 the air-cleaning device connector 41 comprises a number of openings and the second screen cover device holder 51b comprises a corresponding number of hooks. Alternatively, the air-cleaning device connector 41 can comprise for example a magnet, tape, adhesive or Velcro for connection to a corresponding second screen cover device holder 51b. However, if the air-cleaning device connector 41 is tape or adhesive it may not be necessary that the air-cleaning device 1 comprises a second screen cover device holder 51b. The air-cleaning device connector 41 can in that case be connected directly to any suitable surface of the air-cleaning device 1.

Another alternative is shown in Figure 3 where a first end 7a of a disposable screen cover 7 is received in a second screen cover device holder 51b', wherein said second screen cover device holder 51b' comprises a groove 45 provided in the air-cleaning device 1 and a locking member 47 which can be received into the groove 45 and lock the first end 7a of the screen cover 7 between the groove 45 and the locking member 47.

The disposable screen covers 7 are suitably designed to cover at least a substantial part of the air outflow area 9 of the screen 11 of the air-cleaning device 1 and suitably also at least parts of side edges 31 of the air-cleaning device 1. Hereby, by covering a larger part of the air-cleaning device with a sterile screen cover there is less risk that personnel working in the operation room will destroy their sterility by unintentional touching a non-sterile part of the air-cleaning device 1. The screen cover device 3; 3'; 3" is configured to be mounted to the air-cleaning device 1 such that one of the disposable screen covers 7 which is the one to be presently in use covers both at least a substantial part of the air outflow area 9 of the screen 11 of the air-cleaning device 1 and at least parts of side edges 31 of the air-cleaning device 1.

At least some of the disposable screen covers 7 comprise in some embodiments of the invention each a peelable protective layer 33 covering substantially the whole screen cover 7. Substantially the whole screen cover 7 would mean at least 90% of the area of the screen cover 7, but suitably the whole area of the screen cover is covered. In some embodiments the peelable protective layer 33 even projects a few mm outside the screen cover 7 in order to assure sterility. A peelable protective layer 33 is only shown in Figure 1b but peelable protective layers 33 can be provided in all embodiments of the invention. Such a peelable protective layer 33 can suitably be peeled off and disposed just before an operation is started or instruments are prepared on its table. Hereby sterility can be secured. The peelable protective layers 33 may be provided to the disposable screen covers 7 by a suitable adhesive such that they can easily be peeled off without leaving adhesive material on the screen cover 7.

The invention further relates to an air-cleaning device 1 configured to be used together with a screen cover device 3; 3'; 3" as described above in relation to Figures 1-3. Said air-cleaning device 1 comprises a screen 11 with an air outflow area 9 where clean air is flowed out from the air-cleaning device 1 and a first screen cover device holder 51a for holding the first unit 13 of the screen cover device 3; 3'; 3". If the first unit 13 is a roll 13a as described for some embodiments above and the roll comprises a central opening the first screen cover device holder 51a can comprise for example a rod which can be received in the central opening of the roll 13a. The first screen cover device holder 51a can also comprise an enclosing cassette attached to the air-cleaning device 1, into which cassette the first unit 13 can be pushed and whereby the screen covers 7 can be pulled out from the cassette through an opening. Such an enclosing cassette may be suitable when UV sterilization of the screen covers 7 is provided which will be further described below in relation to Figure 3. The first screen cover device holder 51a can also be other types of clamps, magnets, adhesives etc.

The air-cleaning device 1 comprises suitably also a second screen cover device holder 51b. In some embodiments, where the screen cover device 3 comprises a second unit 23, as shown in Figures 1a and 1b, the second screen cover device holder 51b can be similar or identical to the first screen cover device holder 51a. In other embodiments of the invention where only a first unit 13 is provided and each screen cover 7 instead is attached in its first end 7a to the air cleaning device 1 when this screen cover is to be used as shown in Figures 2 and 3 a second screen cover device holder 51b can comprise for example hooks as shown in Figure 2, tape, adhesive, Velcro or magnets or a groove and locking device as shown in Figure 3.

The first and second screen cover device holders 51a, 51b are positioned on the air-cleaning device 1 such that a screen cover 7 can be held in a first end 7a by either a second unit 23 or the second screen cover device holder 51b and in a second end 7b by the first unit 13 while the screen cover 7 is covering at least a substantial part of the air outflow area 9 of the screen 11 of the air-cleaning device 1.

In some embodiments of the invention the air-cleaning device 1 further comprises a germicidal radiation source 61 positioned such that the disposable screen cover to be used can be irradiated. The germicidal radiation source 61 can for example comprise a UVC-source, a LED-source, Ionizing radiation source or a pulsating UV-source. The germicidal radiation source 61 can for example be positioned inside the air-cleaning device 1 and a slit 63 can be provided in a casing of the air-cleaning device 1 such that radiation from the germicidal radiation source 61 can escape out. The first screen cover device holder 51a can be provided close to this slit 63 such that screen covers 7 will be irradiated by the germicidal radiation source 61 when they are removed from the first unit 13. The first screen cover device holder 51a can comprise an enclosing cassette as described above. The enclosing cassette can be designed for not allowing radiation outside which is suitable from a user perspective. The germicidal radiation source 61 can in some embodiments also radiate inwardly into the air-cleaning device 1 such that air to be flowed out through the screen 11 can be irradiated and hereby sterilized. In such an air-cleaning device 1 a HEPA filter may not be necessary or another type of filter may be used.

In another embodiment said germicidal radiation source 61 can be provided to the air-cleaning device 1 such that it can irradiate the disposable screen cover 7 which presently is in use and the air outflow from the air-cleaning device.

In some embodiments of the invention the air-cleaning device 1 further comprises a frame 71 configured to hold the one of the disposable screen covers 7 which is the one to be presently in use in a position such that the disposable screen cover 7 covers at least a substantial part of the air outflow area 9 of the screen 11 of the air-cleaning device 1. The frame 71 comprises in some embodiments one or more pulleys 73 over which the screen covers 7 are running. The frame 71 may also comprise one or more guides 75 which are bent such that the screen cover 7 which is the one presently used is stretched over the guides 75 and provides a curved surface of the screen cover 7. Such guides 75 may be provided one to a top part of the air-cleaning device and one to a bottom part of the air-cleaning device. Hereby the air flow will be guided slightly more towards the sides. Which is advantageous because this will widen the clean zone.

According to the invention an air-cleaning device system comprising an air-cleaning device 1 as described above and a screen cover device 3; 3'; 3" as described above is also provided. The screen cover device 3; 3'; 3" can be connected to the air-cleaning device 1, whereby both the air-cleaning device 1 and the screen cover device 3; 3'; 3" comprises cooperating connecting devices, such as the first and second screen cover device holders 51a, 51b and the air-cleaning device connector 41 as described above.

The disposable screen covers 7 may comprise a weave from for example nylon, cellulose, mesh or non-woven. One part of the screen covers 7 which will cover the air flow out from the screen 11 of the air-cleaning device 1 should be permeable for the air. The rest of the screen covers 7, which are covering at least parts of side edges 31 of the air cleaning device 1 may be covered by a plastic material.

## Claims

1. A screen cover device for holding disposable screen covers (7) which when in use are configured for covering at least a substantial part of an air outflow area (9) of a screen (11) of an air-cleaning device (1), wherein said screen cover device (3; 3'; 3") is provided as an attachment to the air-cleaning device (1) and comprises:
- at least two disposable screen covers (7); and
- a first unit (13) which is configured for keeping the at least two disposable screen covers (7) when unused and which is configured to be mounted to the air-cleaning device (1),
wherein said at least two disposable screen covers (7) are in connection with each other such that they can be removed from the first unit (13) one after the other

2. Screen cover device according to claim 1, wherein the first unit (13) comprises a roll (13a) around which the at least two unused disposable screen covers (7) are rolled.

3. Screen cover device according to any one of the preceding claims, wherein a first end (7a) of each disposable screen cover (7) is configured for being connected to the air-cleaning device (1), whereby a second end (7b) of the disposable screen cover (7) is held by the first unit (13) when this disposable screen cover (7) is the one presently in use covering an air outflow area (9) of a screen (11) of the air-cleaning device (1), which second end (7b) is opposite said first end (7a) of the disposable screen cover (7).

4. Screen cover device according to any one of the claims 1-2, further comprising:
- a second unit (23) for collecting used disposable screen covers (7), which second unit (23) is configured to be mounted to the air-cleaning device (1) such that a screen cover (7) which is the one presently in use for covering at least a substantial part of an air outflow area (9) of a screen (11) of the air-cleaning device (1) is held between the first and the second units (13, 23).

5. Screen cover device according to claim 4, wherein the second unit (23) comprises a roll (23a) around which the used disposable screen covers (7) are rolled.

6. Screen cover device according to claims 2 and 5, wherein the rolls (13a, 23a) of the first and second units (13, 23) are configured such that they can be rotated in coordination for unwinding unused disposable screen covers (7) from the roll (13a) of the first unit (13) and winding used disposable screen covers (7) onto the roll (23a) of the second unit (23).

7. Screen cover device according to any one of the preceding claims, wherein the disposable screen covers (7) are designed to cover at least a substantial part of the air outflow area (9) of the screen (11) of the air-cleaning device (1) and at least parts of side edges (31) of the air-cleaning device (1) and wherein the screen cover device (3; 3'; 3") is configured to be mounted to the air-cleaning device (1) such that one of the disposable screen covers (7) which is the one to be presently in use covers both at least a substantial part of the air outflow area (9) of the screen (11) of the air-cleaning device (1) and at least parts of side edges (31) of the air-cleaning device (1).

8. Screen cover device according to any one of the preceding claims, wherein at least one of the disposable screen covers (7) comprises a peelable protective layer (33).

9. Screen cover device according to any one of the preceding claims, wherein the screen cover device (3; 3'; 3") is presterilized.

10. An air-cleaning device system comprising an air-cleaning device (1) and a screen cover device (3; 3'; 3") according to any one of the claims 1-9, wherein said air-cleaning device (1) comprises:
- a screen (11) with an air outflow area (9) where clean air is flowed out from the air-cleaning device (1); and
- a first screen cover device holder (51a) for holding said first unit (13).

11. Air-cleaning device system according to claim 10, wherein said air-cleaning device (1) further comprises a second screen cover device holder (51b) for holding a second unit (23) of a screen cover device (3; 3'; 3") or for holding a first end (7a) of a screen cover (7).

12. Air-cleaning device system according to claim 11, wherein said second screen cover device holder (51b) is positioned such that a screen cover (7) can be held in a first end (7a) either by a second unit (23) or by the second screen cover device holder (51b) and in a second end (7b) by the first unit (13) while the screen cover (7) is covering at least a substantial part of the air outflow area (9) of the screen (11) of the air-cleaning device (1).

13. Air-cleaning device system according to any one of the claims 10-12, further comprising a germicidal radiation source (61) positioned such that the disposable screen cover (7) to be used can be irradiated.

14. Air-cleaning device system according to claim 13, wherein said germicidal radiation source (61) is provided such that it can irradiate the disposable screen cover (7) which presently is in use and the air outflow from the air-cleaning device.

15. Air-cleaning device system according to any one of the claims 10-14, further comprising a frame (71) configured to hold the one of the disposable screen covers (7) which is the one to be presently in use in a position such that the disposable screen cover (7) covers at least a substantial part of the air outflow area (9) of the screen (11) of the air-cleaning device (1).

16. Air-cleaning device system according to claim 15, wherein the frame (71) comprises guides (75) which are bent such that the screen cover (7) which is the one presently used is stretched over the guides (75) and provides a curved surface of the screen cover.

## Patentansprüche

1. Siebabdeckungsvorrichtung zum Halten von Einweg-Siebabdeckungen (7), die, wenn sie in Gebrauch sind, so konfiguriert sind, dass sie mindestens einen wesentlichen Teil einer Luftauslassfläche (9) eines Siebs (11) einer Luftreinigungsvorrichtung (1) abdecken, wobei die Siebabdeckungsvorrichtung (3; 3'; 3") als Anbauteil an der Luftreinigungsvorrichtung (1) vorgesehen ist und Folgendes umfasst:
- mindestens zwei Einweg-Siebabdeckungen (7); und
- eine erste Einheit (13), die so konfiguriert ist, dass sie die mindestens zwei Einweg-Siebabdeckungen (7), wenn sie unbenutzt sind, aufbewahrt, und die so konfiguriert ist, dass sie an der Luftreinigungsvorrichtung (1) zu montieren ist,
wobei die mindestens zwei Einweg-Siebabdeckungen (7) so miteinander in Verbindung stehen, dass sie nacheinander von der ersten Einheit (13) abgenommen werden können.

2. Siebabdeckungsvorrichtung nach Anspruch 1, wobei die erste Einheit (13) eine Rolle (13a) umfasst, um welche die mindestens zwei unbenutzten Einweg-Siebabdeckungen (7) aufgerollt sind.

3. Siebabdeckungsvorrichtung nach einem der vorstehenden Ansprüche, wobei ein erstes Ende (7a) jeder Einweg-Siebabdeckung (7) dafür konfiguriert ist, mit der Luftreinigungsvorrichtung (1) verbunden zu werden, wodurch ein zweites Ende (7b) der Einweg-Siebabdeckung (7) von der ersten Einheit (13) gehalten wird, wenn diese Einweg-Siebabdeckung (7) die eine ist, die gerade zum Abdecken einer Luftauslassfläche (9) eines Siebs (11) der Luftreinigungsvorrichtung (1) in Benutzung ist, wobei das zweite Ende (7b) dem ersten Ende (7a) der Einweg-Siebabdeckung (7) gegenüberliegt.

4. Siebabdeckungsvorrichtung nach einem der Ansprüche 1-2, weiter umfassend:
- eine zweite Einheit (23) zum Sammeln benutzter Einweg-Siebabdeckungen (7), wobei die zweite Einheit (23) so konfiguriert ist, dass sie an der Luftreinigungsvorrichtung (1) zu montieren ist, sodass eine Siebabdeckung (7), welche die eine ist, die gerade zum Abdecken mindestens eines wesentlichen Teils einer Luftauslassfläche (9) eines Siebs (11) der Luftreinigungsvorrichtung (1) in Benutzung ist, zwischen der ersten und der zweiten Einheit (13, 23) gehalten wird.

5. Siebabdeckungsvorrichtung nach Anspruch 4, wobei die zweite Einheit (23) eine Rolle (23a) umfasst, um welche die benutzten Einweg-Siebabdeckungen (7) aufgerollt sind.

6. Siebabdeckungsvorrichtung nach den Ansprüchen 2 und 5, wobei die Rollen (13a, 23a) der ersten und der zweiten Einheit (13, 23) so konfiguriert sind, dass sie koordiniert gedreht werden können, um unbenutzte Einweg-Siebabdeckungen (7) von der Rolle (13a) der ersten Einheit (13) abzuwickeln und benutzte Einweg-Siebabdeckungen (7) auf die Rolle (23a) der zweiten Einheit (23) aufzuwickeln.

7. Siebabdeckungsvorrichtung nach einem der vorstehenden Ansprüche, wobei die Einweg-Siebabdeckungen (7) so ausgelegt sind, dass sie mindestens einen wesentlichen Teil der Luftauslassfläche (9) des Siebs (11) der Luftreinigungsvorrichtung (1) und mindestens Teile von Seitenkanten (31) der Luftreinigungsvorrichtung (1) abdecken, und wobei die Siebabdeckungsvorrichtung (3; 3'; 3") so konfiguriert ist, dass sie an der Luftreinigungsvorrichtung (1) zu montieren ist, sodass eine der Einweg-Siebabdeckungen (7), welche die eine ist, die gerade in Benutzung ist, sowohl mindestens einen wesentlichen Teil der Luftauslassfläche (9) des Siebs (11) der Luftreinigungsvorrichtung (1) als auch mindestens Teile von Seitenkanten (31) der Luftreinigungsvorrichtung (1) abdeckt.

8. Siebabdeckungsvorrichtung nach einem der vorstehenden Ansprüche, wobei mindestens eine der Einweg-Siebabdeckungen (7) eine abziehbare Schutzschicht (33) aufweist.

9. Siebabdeckungsvorrichtung nach einem der vorstehenden Ansprüche, wobei die Siebabdeckungsvorrichtung (3; 3'; 3") vorsterilisiert ist.

10. Luftreinigungsvorrichtungssystem, umfassend eine Luftreinigungsvorrichtung (1) und eine Siebabdeckungsvorrichtung (3; 3'; 3") nach einem der Ansprüche 1-9, wobei die Luftreinigungsvorrichtung (1) Folgendes umfasst:
- ein Sieb (11) mit einer Luftauslassfläche (9), aus der saubere Luft aus der Luftreinigungsvorrichtung (1) herausströmt; und
- eine erste Siebabdeckungsvorrichtungshalterung (51a) zum Halten der ersten Einheit (13).

11. Luftreinigungsvorrichtungssystem nach Anspruch 10, wobei die Luftreinigungsvorrichtung (1) weiter eine zweite Siebabdeckungsvorrichtungshalterung (51b) zum Halten einer zweiten Einheit (23) einer Siebabdeckungsvorrichtung (3; 3'; 3") oder zum Halten eines ersten Endes (7a) einer Siebabdeckung (7) umfasst.

12. Luftreinigungsvorrichtung nach Anspruch 11, wobei die zweite Halterung für die Siebabdeckung (51b) so angeordnet ist, dass eine Siebabdeckung (7) an einem ersten Ende (7a) entweder von einer zweiten Einheit (23) oder von der zweiten Siebabdeckungsvorrichtungshalterung (51b) und an einem zweiten Ende (7b) von der ersten Einheit (13) gehalten werden kann, während die Siebabdeckung (7) mindestens einen wesentlichen Teil der Luftauslassfläche (9) des Siebs (11) der Luftreinigungsvorrichtung (1) abdeckt.

13. Luftreinigungsvorrichtung nach einem der Ansprüche 10-12, weiter umfassend eine keimtötende Strahlungsquelle (61), die so angeordnet ist, dass die zu benutzende Einweg-Siebabdeckung (7) bestrahlt werden kann.

14. Luftreinigungsvorrichtung nach Anspruch 13, wobei die keimtötende Strahlungsquelle (61) so bereitgestellt wird, dass sie die Einweg-Siebabdeckung (7), die gerade in Benutzung ist, und den Luftauslass aus der Luftreinigungsvorrichtung bestrahlen kann.

15. Luftreinigungsvorrichtung nach einem der Ansprüche 10-14, weiter umfassend einen Rahmen (71), der so konfiguriert ist, dass er die eine der Einweg-Siebabdeckungen (7), welche die eine ist, die gerade in Benutzung ist, so hält, dass die Einweg-Siebabdeckung (7) mindestens einen wesentlichen Teil der Luftauslassfläche (9) des Siebs (11) der Luftreinigungsvorrichtung (1) abdeckt.

16. Luftreinigungsvorrichtungssystem nach Anspruch 15, wobei der Rahmen (71) Führungen (75) umfasst, die so gebogen sind, dass die Siebabdeckung (7), welche die eine ist, die gerade in Benutzung ist, über die Führungen (75) gespannt wird und eine gekrümmte Oberfläche der Siebabdeckung bereitstellt.

## Revendications

1. Dispositif de recouvrement de grille destiné à maintenir des éléments de recouvrement de grille jetables (7) qui, lorsqu'ils sont utilisés, sont configurés pour couvrir au moins une partie substantielle d'une zone de sortie d'air (9) d'une grille (11) d'un dispositif de purification d'air (1), dans lequel ledit dispositif de recouvrement de grille (3 ; 3' ; 3") est fourni comme accessoire au dispositif de purification d'air (1) et comprend :
- au moins deux éléments de recouvrement de grille jetables (7) ; et
- une première unité (13) qui est configurée pour conserver les au moins deux éléments de recouvrement de grille jetables (7) lorsqu'ils ne sont pas utilisés et qui est configurée pour être montée sur le dispositif de purification d'air (1),
dans lequel lesdits au moins deux éléments de recouvrement de grille jetables (7) sont reliés l'un à l'autre de telle sorte qu'ils peuvent être retirés de la première unité (13) l'un après l'autre.

2. Dispositif de recouvrement de grille selon la revendication 1, dans lequel la première unité (13) comprend un rouleau (13a) autour duquel sont enroulés les au moins deux éléments de recouvrement de grille jetables non utilisées (7).

3. Dispositif de recouvrement de grille selon l'une quelconque des revendications précédentes, dans lequel une première extrémité (7a) de chaque élément de recouvrement de grille jetable (7) est configurée pour être connectée au dispositif de purification d'air (1), de sorte qu'une seconde extrémité (7b) de l'élément de recouvrement de grille jetable (7) est maintenue par la première unité (13) lorsque cet élément de recouvrement de grille jetable (7) est celui actuellement utilisé pour recouvrir une zone de sortie d'air (9) d'une grille (11) du dispositif de purification d'air (1), laquelle seconde extrémité (7b) est opposée à ladite première extrémité (7a) de l'élément de recouvrement de grille jetable (7).

4. Dispositif de recouvrement de grille selon l'une quelconque des revendications 1-2, comprenant en outre :
- une seconde unité (23) pour collecter des éléments de recouvrement de grille jetables (7) usagés, laquelle seconde unité (23) est configurée pour être montée sur le dispositif de purification d'air (1) de sorte qu'un élément de recouvrement de grille (7) qui est celui actuellement utilisé pour couvrir au moins une partie substantielle d'une zone de sortie d'air (9) d'une grille (11) du dispositif de purification d'air (1) soit maintenu entre les première et seconde unités (13, 23).

5. Dispositif de recouvrement de grille selon la revendication 4, dans lequel la seconde unité (23) comprend un rouleau (23a) autour duquel les éléments de recouvrement de grille jetables (7) usagés sont enroulés.

6. Dispositif de recouvrement de grille selon les revendications 2 et 5, dans lequel les rouleaux (13a, 23a) des première et seconde unités (13, 23) sont configurés de manière à pouvoir être entraînés en rotation de façon coordonnée pour dérouler des éléments de recouvrement de grille jetables (7) non usagés à partir du rouleau (13a) de la première unité (13) et enrouler des éléments de recouvrement de grille jetables (7) usagés sur le rouleau (23a) de la seconde unité (23).

7. Dispositif de recouvrement de grille selon l'une quelconque des revendications précédentes, dans lequel les éléments de recouvrement de grille jetables (7) sont conçus pour couvrir au moins une partie substantielle de la zone de sortie d'air (9) de la grille (11) du dispositif de purification d'air (1) et au moins des parties de bords latéraux (31) du dispositif de purification d'air (1), et dans lequel le dispositif de recouvrement de grille (3 ; 3' ; 3") est configuré pour être monté sur le dispositif de purification d'air (1) de telle sorte que l'un des éléments de recouvrement de grille jetables (7), celui qui est actuellement utilisé, couvre à la fois au moins une partie substantielle de la zone de sortie d'air (9) de la grille (11) du dispositif de purification d'air (1) et au moins des parties de bords latéraux (31) du dispositif de purification d'air (1).

8. Dispositif de recouvrement de grille selon l'une quelconque des revendications précédentes, dans lequel au moins l'un des éléments de recouvrement de grille jetables (7) comprend une couche protectrice détachable (33).

9. Dispositif de recouvrement de grille selon l'une quelconque des revendications précédentes, dans lequel le dispositif de recouvrement de grille (3 ; 3' ; 3") est préstérilisé.

10. Système de dispositif de purification d'air comprenant un dispositif de purification d'air (1) et un dispositif de recouvrement de grille (3 ; 3' ; 3") selon l'une quelconque des revendications 1-9, dans lequel ledit dispositif de purification d'air (1) comprend :
- un grille (11) avec une zone de sortie d'air (9) où de l'air propre est évacué du dispositif de purification d'air (1) ; et
- un premier support de dispositif de recouvrement de grille (51a) pour maintenir ladite première unité (13).

11. Système de dispositif de purification d'air selon la revendication 10, dans lequel ledit dispositif de purification d'air (1) comprend en outre un second support de dispositif de recouvrement de grille (51b) pour maintenir une seconde unité (23) d'un dispositif de recouvrement de grille (3 ; 3' ; 3") ou pour maintenir une première extrémité (7a) d'un élément de recouvrement de grille (7).

12. Système de dispositif de purification d'air selon la revendication 11, dans lequel ledit second support de dispositif de recouvrement de grille (51b) est positionné de telle sorte qu'un élément de recouvrement de grille (7) puisse être maintenu à une première extrémité (7a) soit par une seconde unité (23), soit par le second support de dispositif de recouvrement de grille (51b), et à une seconde extrémité (7b) par la première unité (13), tandis que l'élément de recouvrement de grille (7) couvre au moins une partie substantielle de la zone de sortie d'air (9) de la grille (11) du dispositif de purification d'air (1).

13. Système de dispositif de purification d'air selon l'une quelconque des revendications 10-12, comprenant en outre une source de rayonnement germicide (61) positionnée de telle sorte que l'élément de recouvrement de grille jetable (7) à utiliser puisse être irradié.

14. Système de dispositif de purification d'air selon la revendication 13, dans lequel ladite source de rayonnement germicide (61) est prévue de manière à pouvoir irradier l'élément de recouvrement de grille jetable (7) qui est actuellement utilisé et la sortie d'air provenant du dispositif de purification d'air.

15. Système de dispositif de purification d'air selon l'une quelconque des revendications 10-14, comprenant en outre un cadre (71) configuré pour maintenir l'un des éléments de recouvrement de grille jetables (7) qui est celui qui doit être actuellement utilisé dans une position telle que l'élément de recouvrement de grille jetable (7) couvre au moins une partie substantielle de la zone de sortie d'air (9) de la grille (11) du dispositif de purification d'air (1).

16. Système de dispositif de purification d'air selon la revendication 15, dans lequel le cadre (71) comprend des guides (75) qui sont pliés de telle sorte que l'élément de recouvrement de grille (7) qui est celui actuellement utilisé soit étiré sur les guides (75) et fournit une surface incurvée de l'élément de recouvrement de grille.
